# EUROPEAN PATENT SPECIFICATION

(11) **EP 2 403 819 B1**
(45) Date of publication and mention of the grant of the patent: **13.08.2014**
(21) Application number: 10709270.2
(22) Date of filing: 25.02.2010
(51) Int. Cl.: C07C 37/00, C07C 37/74, C07C 39/04, C07C 39/06

(54) **PROCESS FOR TREATMENT OF PHENOL AND TAR ACIDS CONTAINING OIL**
VERFAHREN ZUR BEHANDLUNG VON PHENOL UND TEERSÄUREN ENTHALTENDEM ÖL
PROCÉDÉ DE TRAITEMENT DE PÉTROLE CONTENANT DU PHÉNOL ET DES HUILES ACIDES DE GOUDRON

(30) Priority: 03.03.2009 WO PCT/IB2009/000411
(43) Date of publication of application: 11.01.2012
(73) Proprietor: SOLIOS CHEMICAL, 68071 Mulhouse (FR)
(72) Inventor: RIGAUT, Etienne, F-68400 Riedisheim (FR); KLEIBER, Michel, F-68350 Brunstatt (FR); RIOUAL, Céline, F-68490 Chalampe (FR); DIAZ, Javier, Esteban, 68200 Mulhouse (FR)
(74) Representative: Novagraaf Technologies
(86) International application number: PCT/IB2010/000377
(87) International publication number: WO 2010/100536

(56) References cited:
- EP-A- 0 143 472
- DE-B- 1 001 279
- GB-A- 1 509 161
- US-A- 2 888 491
- US-A- 4 469 561

## Description

### ABSTRACT

The following invention relates to a process for recovering phenol and tar acids (cresols, xylenols) from different material feedstocks containing the said phenol and tar acids. The process comprises three to four main units including two cascading liquid-liquid extraction equipment, a solvent recovery unit where a pure blend of phenols (phenol and tar acids) might be obtained, and a purification section where pure phenol and pure isolated tar acids might be obtained by combined crystallizations from the melt and distillation. The primary solvent of the first liquid-liquid extraction unit is water. The secondary solvent in the second liquid-liquid extraction unit is a single water immiscible mono- or polycyclic aromatic hydrocarbon, substituted or not, or a mixture thereof.

### FIELD OF THE INVENTION:

This invention is related to a new process for the treatment of oil containing phenol and tar acids in order to proceed to the recovery of said phenol and tar acids and their purification by combination of liquid-liquid extractions, distillation and further post treatment by combination of crystallizations and distillation.

### DESCRIPTION

Oils containing a mixture of phenols, namely phenol and phenol related compounds (cresols, xylenols), hereafter designed as phenol and tar acids, are obtained by various means such as coal, petroleum or biomass processing. Said oils, or a mix thereof, containing phenol and tar acids resulting from the processing of coal, petroleum or biomass are used as feedstock oils.

The recovery of phenol and tar acids from these oil feedstocks are challenging because of the close distillation range of these compounds and the narrow boiling point range of the oil cuts they are contained in. Direct normal distillation processes are therefore not considered as being suitable for recovering the phenol and tar acids. In the past other methods were investigated noticeably including liquid-liquid extraction systems.

Historical methods used caustic soda to promote the extraction of phenol and tar acids contained in the feedstock oils. As these oils contain mainly basic or neutral groups, the acid character of the phenol and tar acids allow extraction with diluted basic solutions. For this purpose, caustic soda has been used as the solvent in order to produce sodium phenolates. The phenolates are then acidified with the use of some inorganic acid, usually sulfuric acid The advantage for this process is the high extraction of all organic acids contained in the oil, soda acting as a transfer enhancer; the principal drawback is mainly due to important volumes of aqueous waste generation. It is very difficult to re-use the neutralized solution which show high salts content, so important amounts of aqueous waste are generated. In order to avoid this issue, other neutralization agents have been used. Exhaust gases with high carbon dioxide content have been used in order to acidify the phenolate mixture. This solution, while proving efficient for reducing the aqueous waste generation, transfers the problem to the recovery of solid carbonate generated during neutralization. Once the solid has been recovered, it is decomposed in a furnace to produce lime which is later used for caustization of the aqueous phenol solution. As the energy consumption of the oven is important, the resulting efficiency of the process is lowered.

An alternative process developed included liquid-liquid extraction using water as a solvent (US 2,888,491). The extraction of phenol and tar acids was conducted under high temperature (circa 160°C) and pressure (circa 20 barg). It was followed by an important dilution of the aqueous extract with 2 to 6 volumes of diluent for one volume of extract. This dilution was necessary to recover excessive amount of dissolved oil in the extract and was eventually required for insuring acceptable performance of post treatments after stripping of the said diluted extract. But it represented a considerable raise of the volume of phenol and tar acid extract to be further processed and thus results in a lowering of the process efficiency. A finishing purification including an additional liquid-liquid extraction with butylacetate followed by butylacetate recovery and phenol blend concentration closed the process. Several other solvents might be used for the task of phenol recovery from aqueous solutions as many organics achieve the same target: high water immiscibility and favorable distribution of phenol and tar acids toward the solvent. Commercially available systems including the use of ketones (for example MIBK) or ethers have been reported for example for treatment process of phenol rich waste water.

Other processes for phenol and tar acids recovery from oils include advanced liquid-liquid extraction using simultaneous use of solvent, counter solvent and sometime additional diluents (US 5,964,987; US 2,666,796; US 2,766,296; US 2004/0200717). The solvents used are in general glycols (US 5,964,987; US 2,790,834; US 2004/0200717) such as ethylene-glycol or triethylene-glycol. They are used pure or as aqueous solutions. These solvent have various drawbacks such as a rather low selectivity between phenol and other oil compounds; thus their use require the extract to be steam stripped in order to recover excessive oil solubilised in the extraction solvent. Additional drawback is the relative thermal sensitiveness of these solvents: under heat treatment such as during atmospheric distillation, they undergo degradation. It is therefore required to proceed via vacuum distillation for their recovery, which does not necessary completely hinder the degradation risk. As a consequence it requires to withdraw a part of the solvent and to proceed to its partial replacement by means of make up streams.

Other kinds of solvents often used are compounds taken from the alcohol family such as methanol (US 2,666,796; US 2,766,296). Due to volatility of methanol, it is required to compensate the losses with make up fresh solvent. Additionally, methanol is used as an aqueous mixture which requires proceeding to a methanol + water mixture after the solvent recovery prior to its use in a recycle loop. This implies extra processing steps right after methanol recovery which was for example avoided with ethylene glycol.

The counter solvent used are often selected among paraffinic hydrocarbons such as hexane or naphtha cuts (US 5,964,987; US 2,666,796; US 2,766,296; US 2004/0200717). The purpose of using a counter-solvent is to ease the extraction process. The oil to be treated has to be soluble in the counter solvent. This allows improving the efficiency of the liquid-liquid extraction as it modifies the interactions between the mixed oil+counter solvent and extraction solvent. The efficiency of the liquid-liquid extraction process would be dramatically hindered in absence of this counter solvent. This policy however implies that additional recovery loops are required in order to get back the counter solvent. As it is often quite close in nature with the oil compounds with a lower boiling point the recovery may be incomplete and counter solvent losses are likely. The resulting phenol and tar acids extracted oil may therefore be polluted to various extends according to the efficiency of the counter solvent recovery. In the case of counter solvent having a nature quite different form the oil compounds, this will decrease the commercial value of the treated oil because of contamination. This is especially true for feedstocks presenting a high aromatic content such as for coal tar oils in the sense that paraffinic compounds are not naturally predominant compounds of the feedstock.

The preceding systems have proven their importance in the industrial scale, as is proven by the literature references. Yet they remain quite complex and heavy especially in terms of solvent used and recovery loops. The energy consumption is high owing to steam stripping of an extract in some cases and/or additional distillation. They are specifically described for the treatment of concentrated feed stocks presenting for example around 30 wt% of phenol in oil feedstock. They are used for processing important streams of feedstock. The combination of these points make these processes are not necessary competitive for processing of low phenol and tar acid containing oil or small streams of oil as they show optimum investment return and operational expenditure for a given range of capacities and for rich phenol containing oils.

US 2,888,491 relates to a process for the separation of phenols from their mixture with neutral oils comprising the following steps : extraction of phenols with water at a temperature above 100°C, preferably between 150-250°C, under pressure in excess of the vapor pressure of the extraction mixture; dilution of the aqueous extract containing phenols and neutral oils in a additional amount of water, and cooling said extract at a temperature below 100°C, preferably between 60-90°C; distillation with steam; and eventually, extraction of the distillate with an organic solvent, such as butyl acetate.

US 4,469,561 relates to a process for the extraction of phenol and bisphenol A (BPA) from an aqueous solution comprising the following steps : extraction of phenol and BPA with toluene, preferably at a temperature between 20-80°C, more preferably at a temperature between 25-35°C; azeotopic distillation, preferably at a temperature between about 112-180°C and a pressureof 1 atmosphere.

GB 1 509 161 relates to a process for the separation and purification of crude vinylphenol comprising the following steps : extractions of vinylphenol, phenol and homologous compounds (cresols) with an aqueous alkaline solution (for example a sodium hydroxide solution) and a water-insoluble co-solvent chosen among an aromatic hydrocarbon (for example toluene), an ether, an alcohol, a nitro compound, halon, a nitrile, a sulfone, a sulfoxide, and a mixture of at least one of said solvents. EP 0 143 472 relates to a process for the purification of crude phenol comprising the following step: distillation of phenols with added diluted acid and treatment with an oxidizing agent; fractional distillation; and fractional crystallization.

DE 1 001 279 relates to a process for the extraction of phenol and phenol derivatives (cresols, xylenols) from tar comprising the following steps : extraction with water; distillation; crystallization or distillation.

### DISCLOSURE

It is an object of the present invention to reduce the various here above disadvantages by proposing a new set up of liquid-liquid extractions and distillation that may be followed by a crystallization step to produce pure phenol and a tar acids blend and by combined crystallization and distillation steps to produce additional isolated pure tar acids from a phenol and tar acids containing oil.

The present process is therefore meant for the extraction of phenol and tar acids containing oils coming from different feedstocks. These feedstocks can be obtained for example from pyrolysis products as tars from coke production or gasification processes. Other feed stocks form biomass gasification or pyrolysis can be considered too, such as for example oils from pyrolysis of lignocellulosic materials as palm shell.

The present process is declined around three to four main units each one performing the steps set in the following:
- Transferring the acids from the feedstock to the first extraction agent using water as a solvent. The operation is performed for example in a solvent extraction column but is not limited to that kind of equipment.
- The enriched extract coming from the first step is intimately contacted with a water immiscible solvent selected as a single mono- or polycyclic aromatic hydrocarbon, substituted or not, or a mixture of compounds thereof. The water immiscible solvent may be preferably a mono- or polycyclic hydrocarbon substituted by at least one alkyle group, more preferably by at least one methyle and/or ethyle group. For example such water immiscible solvent may be selected in a non limitative way among the following: benzene, toluene, ortho-xylene, meta-xylene, para-xylene, mesitylene, ethylbenzene, 1-methylethylbenzene, 1-ethyl-2-methylbenzene, 1-ethyl-3-methylbenzenenaphthalene, 1,2-dimethyl-3-ethylbenzene, 1,2-dimethyl-4-ethylbenzene, 1,2-dimethyl-5-ethylbenzene, 1,3-dimethyl-2-ethylbenzene, 1,3-dimethyl-4-ethylbenzene, 1,3-dimethyl-5-ethylbenzene, 1,4-dimethyl-2-ethylbenzene, naphthalene, 1-methylnaphthalene, 2-methylnaphthalene, 1-ethylnaphthalene, 2-ethylnaphthalene, 1,2 dimethylnaphthalene, 1,6 dimethylnaphtalene, biphenyl, 2,6 dimethylnaphthalene, indene, fluoranthene, anthracene, phenanthrene, or a mixture of compounds thereof and various traces of other hydrocarbons thereof noticeably including toluol, benzol, BTX or wash-oil industrial distillation cuts or blends.
- In a third step, the secondary solvent and the extracted phenol and tar acids are separated via common distillation, producing a concentrated blend of phenol and tar acids and a refined solvent on top of the column.
- Finally, the blend coming from distillation sump is separated via crystallization(s) to produce pure phenol and a tar acids blend. Crystallization is more efficient for effective pure phenol and tar acids recovery than previously claimed accurate fractionation distillation processes.

In a preferred embodiment the present process is carried out without final crystallization unit. This is generally the case when low or intermediate volumes of phenol and tar acids containing oils are treated, and the concentrated blend of phenol and tar acids may be directly used in this form or further processed by the user.

In a preferred embodiment the crystallization step is completed by crystallizations combined with distillations to produce additional isolated pure tar acids.

The present invention represents the following advantages and improvements:
- It does not require the use of any base or acid that usually lead to the production of substantial amount of waste material to be processed.
- It is adapted for competitive processing of oil feedstocks presenting low initial load in phenol and tar acids.
- It is adapted for competitive processing of low and oil feed rates.
- The solvents used allow a selective removal of phenol with limited or negligible oil uptake.
- The solvents used do not show thermo-sensitive behaviour and can be recovered under optimum conditions ensuring a limited extend of make-up and drain losses.
- The solvents used are selected accordingly to the feed composition and do not counter fake the commercial value of the treated oil produced as they do not exhibit any contaminative character.
- There is no production of residual solid wastes such as sodium carbonate or sodium sulphate.

The present invention represents the following distinguish features:
- Two cascading liquid-liquid extraction systems.
- Water used as first solvent.
- A single mono- or polycyclic aromatic hydrocarbon, substituted or not, or a mixture of compounds thereof used as second solvent.
- No need of stripping for dissolved oil recovery.
- Final purification by crystallization(s) and/or distillation.

The present invention shall now be described in detail, in a non-limiting guideline example, with special reference to the enclosed figure, where:

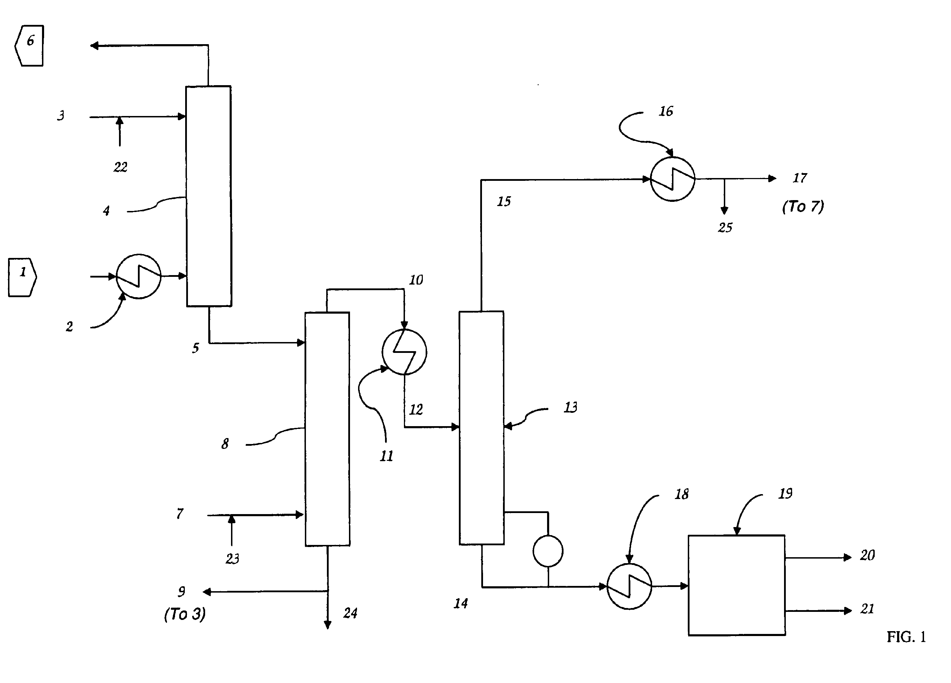

FIG. 1 schematically illustrates a treatment plant for pure phenol and tar acids blend production from a phenol and tar acids containing oil according to the invention.

The feed stock of phenol and tar acids containing oil coming from stream **1** is set at the required temperature for optimized recovery by mean of heat exchanger **2.** The target temperature is set between 60 and 160°C under a pressure in excess of the vapour pressure of the mixture formed. The feed stock is then introduced in the liquid-liquid extraction equipment **4** where it is treated with water. The water is introduced via stream **3.** The stream **3** is recovered from the stream **9** (described later). Water make up can be made through stream **22.** The extraction done in the extraction equipment **4** results in a treated oil stream **6** and an aqueous phenol and tar acids solution **5.** The treated oil **6** is substantially free of phenol. The stream **6** can be, according to its composition, subject to further purification treatment, for example by distillation or crystallization from the melt.

The aqueous phenol and tar acids solution **5** is then introduced in the liquid-liquid extraction equipment **8** to be processed with an hydrocarbon solvent chosen among single mono- or polycyclic aromatic hydrocarbons, substituted or not, or a mixture of compounds thereof. The hydrocarbon solvent is introduced in the liquid-liquid extraction equipment **8** through stream **7.** The stream **7** is recovered form the stream **17** (described later). Hydrocarbon solvent make up can be made through stream **23.** The extraction done in the extraction equipment **8** results in two streams: a water stream **9** and an organic phenol and tar acids solution **10.** The water stream **9** is substantially free of phenol and tar acids. The possibility is left to withdraw a part of the water from stream **9** by mean of drain stream **24.**

The organic phenol and tar acids solution **10** is heated up to the required temperature by means of pre-heater **11.** The pre-heater **11** is required to set the feed stream **12** of the distillation **13** at the required temperature for adapted distillation management. The heating medium used in pre-heater **11** can be a specific utility stream (not shown) or be designed within the frame of an optimized heat management of the plant by means of additional heat exchangers or economizers for example. The pre-heater **11** therefore represents a function rather than single equipment.

The pre-heated organic phenol and tar acids solution **10** is introduced in the distillation system **13. 13** is a solvent recovery system that allows recovering the hydrocarbon solvent as an overhead product. The distillation **13** is operated at a pressure allowing optimized recovery of the hydrocarbon solvent.

The hydrocarbon solvent is recovered as stream **15** and condensed in heat exchanger **16.** The cooling medium used in condenser **16** can be a specific utility stream (not shown) or be designed within the frame of an optimized heat management of the plant by means of additional heat exchangers or economizers for example. The condenser **16** therefore represents a function rather than single equipment. The condensed and cooled down hydrocarbon solvent is recovered as stream **17** and recycled to stream **7.** The possibility is left to withdraw a part of the hydrocarbon solvent from stream **17** by mean of stream **25.**

A concentred blend of phenol and tar acids is recovered as stream **14** at the sump of distillation equipment **13.** It is directed to the heat exchanger **18.** The purpose of the heat exchanger **18** is to cool down the concentrated blend of phenol and tar acids coming from distillation **13** prior to feed it to the crystallization unit **19.** The cooling medium used in cooler **18** can be a specific utility stream (not shown) or be designed within the frame of an optimized heat management of the plant by means of additional heat exchangers or economizers for example. The condenser **18** therefore represents a function rather than single equipment.

The section **19** depicts the last purification stage leading to the production of phenol and a blend of tar acids. The section **19** consists in one or more crystallization equipment eventually combined with additional distillation systems. The detailed nature and the arrangement of the section **19** are determined in function of the purification policy selected. The purification policy will rely on the initial repartition of phenol and various tar acids in the initial load **1.** It will lead to the production of phenol and a blend of tar acids or phenol and a blend of tar acids plus fractionated isolated tar acids. The section **19** can be operated to allow phenol recovery with a purity superior to 95% or preferably as a pure phenol with a purity superior to 99%. This is depicted through streams **20** and **21** in the specific case of production of a pure phenol stream **20** and a blend of tar acids **21.**

### EXAMPLE

The following example describes a plant as per previous description meant for extracting phenol and tar acids from a 1500 kg/h stream of oil feedstock. The preferred embodiment of the process is the one shown in the drawing of Figure 1 without final crystallization unit.

An inlet oil feedstock **1** containing 10 to 20% phenol and tar acids is fed to heat exchanger **2.** The distribution coefficient for phenol and tar acids in the oil/water system is favoured at higher temperatures. In heat exchanger **2** the temperature is adjusted to the conditions of the column between 60 and 160°C as stated earlier. A temperature of 90°C is preferred for the given example. The exit stream of heat exchanger **2** is then fed to liquid-liquid extraction equipment **4** where it is counter-currently contacted with water. In the present example the extraction equipment **4** used is a solvent extraction column with a pulsating device and selected sieve tray internals. Other technologies of equipment may be used. The operating pressure in the column **4** is in excess of the vapour pressure of the mixture formed within in order not boil the mixture.

The water used in equipment **4** is coming from stream **3** and water make up stream **22.** As the water coming from stream **3** is recycled from stream **9,** the mass flow ratio of stream **3** to **22** is greater than 30. The solvent to feed ratio in the equipment **4** must be greater to 6 in order to extract the so told percentage of phenol and tar acids contained in the oil feedstock. The water solvent is such that the initial oil is freed from its phenol and tar acids content in more than 95% and that an acid content in the exit stream **6** of less than 5% is obtained.

The stream 5 is then feed in the liquid-liquid extraction equipment **8** where it is treated with water-immiscible solvent. This second liquid-liquid extraction equipment **8** is in the present example of the same nature than **4,** with pulsing agitation device and counter-current flow of feed and solvent streams. Other technologies of equipment may be used. It is noteworthy that the parameters for mixing, the nature and arrangement of internals will differ from equipment **4** to equipment **8.**

In the column **8,** the aqueous solution of stream 5 is counter-currently extracted with an immiscible organic solvent selected from a single mono- or polycyclic aromatic hydrocarbon, substituted or not, or a mixture of compounds thereof. In the present example the solvent is toluene. The operating pressure in the column **8** is in excess of the vapour pressure of the mixture formed within in order not boil the mixture. The outlets of column **8** are on one hand, the rich acid aromatic extract **10,** and on the other hand, the primary solvent (water) stream **9** for reuse in the first column **4.** Due to extremely low solubility of the selected aromatic compound in the aqueous solvent, clear separation of the phases is obtained and there is no need for special devices for coalescence. The toluene fed by stream 7 to this column comes mainly from the condensed and cooled down hydrocarbon solvent recycled from stream **17** to stream **7.** Make-up can be done through stream **23.** The recovered water exit stream **9** is recycled to the column **4** through stream **3.** The water stream **9** is substantially free of phenol and tar acids. In the current example less than 900 ppm were obtained.

The organic stream **10** recovered at exit of column **8** is preheated by heat exchanger **11** to a temperature around 120°C, i.e. close to the bubble temperature of the said mixture. The composition of this stream is no higher than 10 wt% in phenol and tar acids.

The pre-heated mixture **12** is fed in the distillation column 13. On top of the column **13** nearly pure secondary solvent is recovered and the bottoms **14** are constituted mainly of tar acids, with toluene in less than 10 wt% and some traces of other extractable compounds contained in the initial feed stock oil. In the present example the distillation column is operated at atmospheric pressure.

The secondary solvant is recovered as stream **15** as distillation overheads. It is condensed in condenser **16** and cooled down to the required temperature to be fed back as a recycled solvent in column **8** through the streams **17** and **7.**

Finally, the tar acids blend produced at the distillation sump **14** is fed to a melt static crystallization unit **19.** The crystallization process was a ProABD^{®} MSC process. Pure phenol product was obtained by crystallization with a purity superior to 99% at stream **20.** The rest of the tar acids contained in the initial blend **14** where recovered with some phenol and other traces of the extractable compounds in stream **21.**

## Claims

1. A process for producing phenol and tar acids from an oil comprising the steps of:
i) Subjecting the oil to a liquid-liquid extraction using a solvent in the form of water to extract the oil contained phenol and tar acids as a phenol and tar acids containing aqueous solution and recovering a treated oil.
ii) Subjecting the said phenol and tar acids containing aqueous solution to a liquid-liquid extraction using a solvent in the form of an hydrocarbon consisting in a single water immiscible mono- or polycyclic aromatic hydrocarbon, or a mixture of compounds thereof, to extract the phenol and tar acids from said phenol containing aqueous solution and to recover a phenol and tar acids lean water and a phenol containing hydrocarbon.
iii) Subjecting the said phenol and tar acids containing hydrocarbon to distillation below, at or above atmospheric pressure resulting in the purification and recovery of said hydrocarbon as an overhead product
iv) Recovering the phenol and tar acids from the bottom products of said distillation as a concentrated blend of phenol and tar acids substantially free of hydrocarbon and water.
v) Recycling the said phenol and tar acids lean water to the liquid-liquid extraction of step (i)
vi) Recycling the said hydrocarbon recovered from step (iii) to the liquid-liquid extraction of step (ii).

2. The process according to claim 1, wherein the hydocarbon of step ii) consists in a water immiscible mono- or polycyclic aromatic hydrocarbon substituted by at least one alkyle group, or a mixture of compounds thereof.

3. The process according to claim 2, wherein the hydrocarbon is selected in the group consisting of benzene, toluene, ortho-xylene, meta-xylene, para-xylene, mesitylene, ethylbenzene, 1-methylethylbenzene, 1-ethyl-2-methylbenzene, 1-ethyl-3-methylbenzenenaphthalene, 1,2-dimethyl-3-ethylbenzene, 1,2-dimethyl-4-ethylbenzene, 1,2-dimethyl-5-ethylbenzene, 1,3-dimethyl-2-ethylbenzene, 1,3-dimethyl-4-ethylbenzene, 1,3-dimethyl-5-ethylbenzene, 1,4-dimethyl-2-ethylbenzene, naphthalene, 1-methylnaphthalene, 2-methylnaphthalene, 1-ethylnaphthalene, 2-ethylnaphthalene, 1,2 dimethylnaphthalene, 1,6 dimethylnaphtalene, biphenyl, 2,6 dimethylnaphthalene, indene, fluoranthene, anthracene, phenanthrene, or a mixture of compounds thereof.

4. The process according to claim 1 further comprising the step of:
vii) Subjecting the said concentrated blend of phenol and tar acids to a purification using at least one step of crystallization from the melt to recover pure phenol and a blend of tar acids.

5. The process according to claim 1 further comprising the step of:
vii) Subjecting the said concentrated blend of phenol and tar acids to a purification using a combination of at least one crystallization from the melt and at least one distillation to recover fractionated compounds composing the tar acids including phenol.

6. The process according to claim 5 wherein the purification of step vii) is carried out using a combination of two cristallizations from the melt and one distillation to recover fractionated compounds composing the tar acids including phenol.

7. The process according to any of claims 1 to 6 wherein the liquid-liquid extractions at steps (i) and (ii) are operated between 60 and 160°C and under a pressure in excess of the vapour pressure of the mixture formed.

8. The process according to any of claims 1 to 7 wherein said oil is a feedstock oil containing phenol and tar acids.

9. The process according to any of claims 1 to 8 wherein the solvent in the form of a hydrocarbon is toluene.

## Patentansprüche

1. Verfahren zur Herstellung von Phenol und Teersäuren aus einem Öl, bei dem man
i) das Öl einer Flüssig-Flüssig-Extraktion unter Verwendung eines Lösungsmittels in Form von Wasser zur Extraktion des in dem Öl enthaltenen Phenols und der in dem Öl enthaltenen Teersäuren in Form einer Phenol und Teersäuren enthaltenden wässrigen Lösung unterwirft und ein behandeltes Öl gewinnt,
ii) die Phenol und Teersäuren enthaltende wässrige Lösung einer Flüssig-Flüssig-Extraktion unter Verwendung eines Lösungsmittels in Form eines Kohlenwasserstoffs, der aus einem einzigen nicht mit Wasser mischbaren mono- oder polycyclischen aromatischen Kohlenwasserstoff oder einem Gemisch von Verbindungen davon besteht, zur Extraktion des Phenols und der Teersäuren aus der Phenol enthaltenden wässrigen Lösung und zur Gewinnung eines an Phenol und Teersäuren armen Wassers und eines Phenol enthaltenden Kohlenwasserstoffs unterwirft,
iii) den Phenol und Teersäuren enthaltenden Kohlenwasserstoff einer Destillation unter, bei oder über Atmosphärendruck unterwirft, was zur Reinigung und Rückgewinnung des Kohlenwasserstoffs als Kopfprodukt führt,
iv) das Phenol und die Teersäuren aus den Sumpfprodukten der Destillation in Form einer konzentrierten Mischung von Phenol und Teersäuren, die weitgehend frei von Kohlenwasserstoff und Wasser ist, gewinnt,
v) das an Phenol und Teersäuren arme Wasser in die Flüssig-Flüssig-Extraktion von Schritt (i) zurückführt,
vi) den aus Schritt (iii) zurückgewonnenen Kohlenwasserstoff in die Flüssig-Flüssig-Extraktion von Schritt (ii) zurückführt.

2. Verfahren nach Anspruch 1, bei dem der Kohlenwasserstoff von Schritt ii) aus einem nicht mit Wasser mischbaren mono- oder polycyclischen aromatischen Kohlenwasserstoff, der durch mindestens eine Alkylgruppe substituiert ist, oder einem Gemisch von Verbindungen davon besteht.

3. Verfahren nach Anspruch 2, bei dem man den Kohlenwasserstoff aus der Gruppe bestehend aus Benzol, Toluol, ortho-Xylol, meta-Xylol, para-Xylol, Mesitylen, Ethylbenzol, 1-Methylethylbenzol, 1-Ethyl-2-methylbenzol, 1-Ethyl-3-methylbenzolnaphthalin, 1,2-Dimethyl-3-ethylbenzol, 1,2-Dimethyl-4-ethylbenzol, 1,2-Dimethyl-5-ethylbenzol, 1,3-Dimethyl-2-ethylbenzol, 1,3-Dimethyl-4-ethylbenzol, 1,3-Dimethyl-5-ethylbenzol, 1,4-Dimethyl-2-ethylbenzol, Naphthalin, 1-Methylnaphthalin, 2-Methylnaphthalin, 1-Ethylnaphthalin, 2-Ethylnaphthalin, 1,2-Dimethylnaphthalin, 1,6-Dimethylnaphthalin, Biphenyl, 2,6-Dimethylnaphthalin, Inden, Fluoranthen, Anthracen, Phenanthren oder einem Gemisch von Verbindungen davon auswählt.

4. Verfahren nach Anspruch 1, bei dem man ferner:
vii) die konzentrierte Mischung von Phenol und Teersäuren einer Reinigung unter Verwendung mindestens eines Schritts der Kristallisation aus der Schmelze zur Gewinnung von reinem Phenol und einer Mischung von Teersäuren unterwirft.

5. Verfahren nach Anspruch 1, bei dem man ferner:
vii) die konzentrierte Mischung von Phenol und Teersäuren einer Reinigung unter Verwendung einer Kombination von mindestens einer Kristallisation aus der Schmelze und mindestens einer Destillation zur Gewinnung von fraktionierten Verbindungen, die sich aus den Teersäuren einschließlich Phenol zusammensetzen, unterwirft.

6. Verfahren nach Anspruch 5, bei dem man die Reinigung von Schritt vii) unter Verwendung einer Kombination von zwei Kristallisationen aus der Schmelze und einer Destillation zur Gewinnung von fraktionierten Verbindungen, die sich aus den Teersäuren einschließlich Phenol zusammensetzen, durchführt.

7. Verfahren nach einem der Ansprüche 1 bis 6, bei dem man die Flüssig-Flüssig-Extraktionen in den Schritten (i) und (ii) zwischen 60 und 160°C und unter einem Druck, der über dem Dampfdruck des gebildeten Gemischs liegt, betreibt.

8. Verfahren nach einem der Ansprüche 1 bis 7, bei dem es sich bei dem Öl um ein Phenol und Teersäuren enthaltendes Einsatzstoff-Öl handelt.

9. Verfahren nach einem der Ansprüche 1 bis 8, bei dem es sich bei dem Lösungsmittel in Form eines Kohlenwasserstoffs um Toluol handelt.

## Revendications

1. Procédé pour la production de phénol et d'acides de goudron à partir d'une huile comprenant les étapes consistant à :
i) soumettre l'huile à une extraction liquide-liquide en utilisant un solvant sous forme aqueuse pour extraire le phénol et les acides de goudron contenus dans l'huile sous forme d'une solution aqueuse contenant le phénol et les acides de goudron et récupérer l'huile traitée.
ii) soumettre ladite solution aqueuse contenant le phénol et les acides de goudron à une extraction liquide-liquide utilisant un solvant sous la forme d'un hydrocarbure consistant en un seul hydrocarbure aromatique mono- ou polycyclique non miscible dans l'eau, ou un mélange de composés de celui-ci, pour extraire le phénol et les acides de goudron de ladite solution aqueuse contenant ledit phénol et récupérer une eau pauvre en phénol et en acides de goudron et un hydrocarbure contenant le phénol.
iii) soumettre ledit hydrocarbure contenant le phénol et les acides de goudron à une distillation en-dessous, à ou au-dessus de la pression atmosphérique résultant en la purification et la récupération dudit hydrocarbure en tant que produit de tête.
iv) récupérer le phénol et les acides de goudron à partir des produits de fond de ladite distillation sous forme d'un mélange concentré de phénol et d'acides de goudron sensiblement exempt d'hydrocarbure et d'eau.
v) recycler ladite eau pauvre en phénol et en acides de goudron vers l'étape d'extraction liquide-liquide de l'étape i).
vi) recycler ledit hydrocarbure récupéré de l'étape iii) vers l'étape d'extraction liquide-liquide de l'étape ii).

2. Procédé selon la revendication 1, où l'hydrocarbure de l'étape ii) consiste en un hydrocarbure aromatique mono- ou polycyclique non miscible dans l'eau substitué par au moins un groupement alkyle, ou un mélange de composés de celui-ci.

3. Procédé selon la revendication 2, où l'hydrocarbure est sélectionné dans le groupe constitué du benzène, toluène, ortho-xylène, méta-xylène, paraxylène, mésitylène, éthylbenzène, 1-méthyléthylbenzène, 1-éthyl-2-méthylbenzène, 1-éthyl-3-méthylbenzènenaphthalène, 1,2-diméthyl-3-éthylbenzène, 1,2-diméthyl-4-éthylbenzène, 1,2-diméthyl-5-éthylbenzène, 1,3-diméthyl-2-éthylbenzène, 1,3-diméthyl-4-éthylbenzène, 1,3-diméthyl-5-éthylbenzène, 1,4-diméthyl-2-éthylbenzène, naphthalène, 1-méthylnaphthalène, 2-méthylnaphthalène, 1-éthylnaphthalène, 2-éthylnaphthalène, 1,2-diméthylnaphthalène, 1,6-diméthylnaphthalène, biphényl, 2,6-diméthylnaphthalène, indène, fluoranthène, anthracène, phénanthrène, ou un mélange des composés de ceux-ci.

4. Procédé selon la revendication 1 comprenant en outre l'étape consistant à :
vii) soumettre ledit mélange concentré de phénol et d'acides de goudron à une purification utilisant au moins une étape de cristallisation de la masse fondue pour récupérer du phénol pur et un mélange d'acides de goudron.

5. Procédé selon la revendication 1 comprenant en outre l'étape consistant à :
vii) soumettre ledit mélange concentré de phénol et d'acides de goudron à une purification utilisant une combinaison d'au moins une cristallisation de la masse fondue et au moins une distillation pour récupérer les composés fractionnés composant le phénol incluant les acides de goudron.

6. Procédé selon la revendication 5 où la purification de l'étape vii) est réalisée en utilisant une combinaison de deux cristallisations de la masse fondue et une distillation pour récupérer les composés fractionnés composant le phénol incluant les acides de goudron.

7. Procédé selon l'une quelconque des revendications 1 à 6 où les extractions liquide-liquide aux étapes i) et ii) sont réalisées entre 60 et 160°C et sous une pression supérieure à la pression de vapeur du mélange formé.

8. Procédé selon l'une quelconque des revendications 1 à 7 où ladite huile est une huile de charge d'alimentation contenant le phénol et les acides de goudron.

9. Procédé selon l'une quelconque des revendications 1 à 8 où le solvant sous la forme d'un hydrocarbure est le toluène.
